# EUROPÄISCHE PATENTSCHRIFT

(11) **EP 0 882 075 B1**
(45) Veröffentlichungstag und Bekanntmachung des Hinweises auf die Patenterteilung: **10.11.1999**
(21) Anmeldenummer: 97903354.5
(22) Anmeldetag: 21.02.1997
(51) Int. Cl.: C08F 8/32, C08F 8/30, C08F 8/00, C10L 1/22, C10M 133/52, C07C 235/80, C07C 255/25, C10M 133/24, C10M 133/16

(54) **KRAFT- UND SCHMIERSTOFFADDITIVE**
FUEL AND LUBRICANT ADDITIVES
ADDITIFS POUR CARBURANTS ET LUBRIFIANTS

(30) Priorität: 23.02.1996 DE 19606845; 23.02.1996 DE 19606846
(43) Veröffentlichungstag der Anmeldung: 09.12.1998
(73) Patentinhaber: BASF AKTIENGESELLSCHAFT, 67056 Ludwigshafen (DE)
(72) Erfinder: JULIUS, Manfred, D-67117 Limburgerhof (DE); ETTL, Roland, D-67454 Ha loch (DE); GÜNTHER, Wolfgang, D-67582 Mettenheim (DE); GREINDL, Thomas, D-67098 Bad Dürkheim (DE)
(74) Vertreter: Kinzebach, Werner, Dr.
(86) Internationale Anmeldenummer: EP9700849
(87) Internationale Veröffentlichungsnummer: WO9731037

(56) Entgegenhaltungen:
- EP-A- 0 568 873
- EP-A- 0 698 598
- DE-B- 1 142 859
- FR-A- 2 333 853
- FR-A- 2 368 467
- GB-A- 1 162 175
- GB-A- 1 378 948
- GB-A- 2 247 457
- US-A- 4 551 526
- US-A- 4 839 073
- US-A- 5 319 126

## Beschreibung

Die vorliegende Erfindung betrifft neue derivatisierte Polyalkylamin-Additive für Kraft- und Schmierstoffe, Verfahren zur Herstellung dieser Additive und Kraft- und Schmierstoffe sowie Additivkonzentrate, welche diese neuartigen Additive enthalten.

Vergaser und Einlaßsysteme von Ottomotoren, aber auch Einspritzsysteme für die Kraftstoffdosierung in Otto- und Dieselmotoren werden in zunehmendem Maße durch Verunreinigungen belastet. Die Verunreinigungen werden verursacht durch Staubteilchen aus der vom Motor angesaugten Luft, unverbrannte Kohlenwasserstoffreste aus dem Brennraum und die in den Vergaser geleiteten Entlüftungsgase aus dem Kurbelwellengehäuse.

Diese Rückstände verschieben das Luft-Kraftstoffverhältnis im Leerlauf und im unteren Teillastbereich, so daß das Gemisch fetter und die Verbrennung unvollständiger wird. Als Folge davon erhöht sich der Anteil unverbrannter oder teilverbrannter Kohlenwasserstoffe im Abgas und der Benzinverbrauch steigt.

Es ist bekannt, daß zur Vermeidung dieser Nachteile Kraftstoffadditive zur Reinhaltung von Ventilen und Vergaser- bzw. Einspritzsystemen verwendet werden (vgl. z.B.: M. Rossenbeck in Katalysatoren, Tenside, Mineralöladditive, Hrsg. J. Falbe, U. Hasserodt, S. 223, G. Thieme Verlag, Stuttgart 1978). Je nach Wirkungsweise und bevorzugtem Wirkort solcher Detergens-Additive unterscheidet man heute zwei Generationen. Die erste Additiv-Generation konnte nur die Bildung von Ablagerungen im Ansaugsystem verhindern, nicht aber bereits vorhandene Ablagerungen wieder entfernen. Die Additive der zweiten Generation können dagegen Ablagerungen verhindern und beseitigen (keep-clean- und clean-up-Effekt). Dies wird insbesondere durch deren hervorragende Thermostabilität an Zonen höherer Temperatur, wie insbesondere an den Einlaßventilen, ermöglicht.

Das molekulare Bauprinzip dieser als Detergenzien wirkenden Additive der zweiten Generation beruht auf der Verknüpfung polarer Strukturen mit meist höhermolekularen, unpolaren oder oleophilen Resten. Typische Vertreter der zweiten Additiv-Generation sind Produkte auf der Basis von Polyisobuten im unpolaren Molekülteil, wie insbesondere Additive vom Polyisobutylamin-Typ.

Derartige Detergenzien sind, ausgehend von Polyisobutenen, nach zwei verschiedenen mehrstufigen Syntheseverfahren herstellbar.

Das erste Verfahren verläuft über eine Chlorierung des polymeren Grundkörpers, gefolgt von einer nucleophilen Substitution des polymeren Grundkörpers durch Amine oder bevorzugt Ammoniak. Nachteilig bei diesem Verfahren ist die Verwendung von Chlor, die zur Folge hat, daß chlor- oder chloridhaltige Produkte auftreten, was heute keinesfalls mehr erwünscht ist.

Im zweiten Verfahren werden die Polyisobutylamine ausgehend von Polyisobuten über Hydroformylierung und anschließend reduktive Aminierung gemäß EP 0 244 616 hergestellt.

Wird in diesem zweiten Verfahren bei der reduktiven Aminierung Ammoniak eingesetzt, so zeigen die Umsetzungsprodukte des Ammoniaks meist eine ausgezeichnete Wirksamkeit bezüglich Ventil- und Vergaserreinhaltung, verhalten sich jedoch allenfalls neutral in ihrer Wirkung auf einen Motorschmierstoff, insbesondere hinsichtlich ihrer Ölschlamm dispergierenden Wirkung.

Von besonderem technischen und wirtschaftlichen Vorteil sind daher solche Additive, die gleichzeitig die Eigenschaften von Detergenzien und Dispergatoren in sich vereinigen und über einfache, chlorfreie Syntheseverfahren erhältlich sind.

Solche Additive sind aus der EP 0 568 873 bekannt. Darin werden β-Aminonitrile der Formel 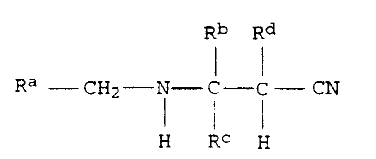 beschrieben, worin
- R^{a}: einen aliphatischen, Alkyl-Seitengruppen aufweisenden Kohlenwasserstoffrest mit einem Molekulargewicht (Zahlenmittel) von 250 bis 5 000 darstellt und
- R^{b}, R^{c} und R^{d}: unabhängig voneinander Wasserstoff oder einen C₁-C₈-Alkylrest oder R^{b} oder R^{d} einen Phenylrest bedeuten, sowie die durch Hydrierung erhaltenen entsprechenden N-Alkyl-1,3-propylendiamine.

Es besteht jedoch ein zusätzlicher Bedarf an weiteren chlorfreien Additivkomponenten, um die Additivzusätze den Anforderungen des jeweiligen Anwendungsgebiets besser anpassen zu können.

Der vorliegenden Erfindung liegt deshalb die Aufgabe zugrunde, weitere, als Kraft- und Schmierstoffadditive geeignete, über chlorfreie Synthese zugängliche Verbindungen zur Verfügung zu stellen.

Es wurde nun gefunden, daß diese Aufgabe gelöst wird durch Bereitstellung von Derivaten langkettiger Amine, die man über eine Funktionalisierung der Amingruppe durch Cyanmethylierung oder durch Umsetzung mit Diketenen erhält.

Eine Cyanmethylierung im Sinne der vorliegenden Erfindung umschreibt die Anbindung einer Cyanoalkylgruppe über deren α-Kohlenstoffatom an einen Amin-Stickstoff.

Gegenstand der vorliegenden Erfindung sind insbesondere Verbindungen der allgemeinen Formel I

Z―CH₂―NY₂₋ₙHₙ (I)

worin
- n: für 0 oder 1 steht;
- Z: für einen geradkettigen oder verzweigten Polyalkylrest mit einem zahlenmittleren Molekulargewicht von 500 bis 40000 steht;
- Y: für einen Rest der Formel IIa oder IIb 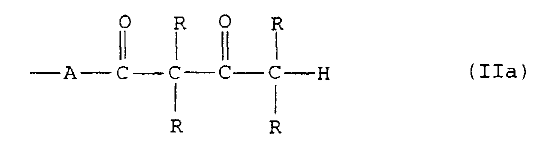 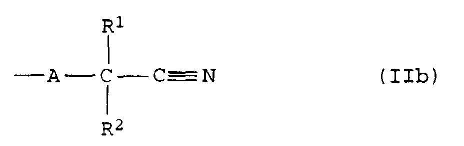 steht, worin
R, R¹ und R² gleich oder verschieden sind und unabhängig voneinander ausgewählt sind unter einem Wasserstoffatom, gegebenenfalls substituierten Alkyl-, Alkenyl-, Alkinylresten, und gegebenenfalls substituierten Cycloalkyl-, Aryl- oder Arylalkylresten, die gegebenenfalls ein oder mehrere Heteroatome aufweisen,
A für einen Alkyleniminrest der Formel III 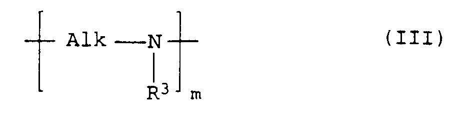 steht, worin
m für einen ganzzahligen Wert von 0 bis 10 steht;
Alk für einen geradkettigen oder verzweigten, gegebenenfalls substituierten Alkylenrest steht,
R³ für ein Wasserstoffatom, einen Alkyl-, Alkenyl-, Alkinyl- oder einen Arylrest steht oder, wenn Y für einen Rest der Formel IIa steht, für einen Ketorest der Formel IVa 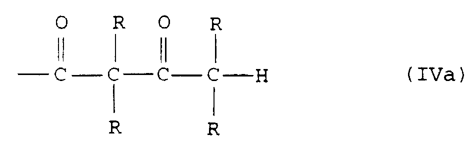 steht, worin die Reste R die oben angegebenen Bedeutungen besitzen,
oder, wenn Y für einen Rest der Formel IIb steht, für einen Cyanorest der Formel IVb 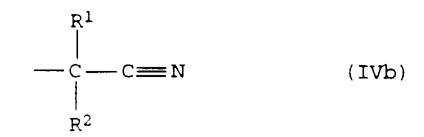 steht, worin R¹ und R² die oben angegebenen Bedeutungen besitzen; oder,
wenn n für 0 steht, einer der Reste Y gegebenenfalls für einen Polyoxyalkylenrest der Formel V 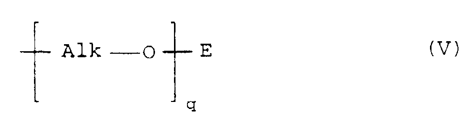 steht, worin
q für eine ganze Zahl von 1 bis 30 steht,
Alk wie oben definiert ist, und
E für ein Wasserstoffatom oder einen C₁-C₆-Alkylrest steht.

Gemäß einer ersten bevorzugten Ausführungsform der Erfindung werden cyanmethylierte Verbindungen der Formel I bereitgestellt, worin Z und n die oben angegebenen Bedeutungen besitzen und Y für einen Rest der Formel IIc 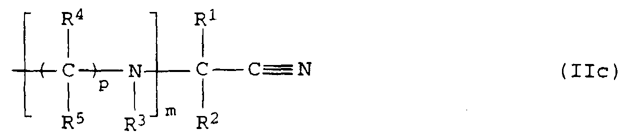 steht, worin
- R¹, R², R³ und m: die oben angegebenen Bedeutungen besitzen,
- R⁴ und R⁵: gleich oder verschieden sind und die oben für R¹ und R² angegebenen Bedeutungen besitzen; und
- p: für einen ganzzahligen Wert von 2 bis 5 steht.

Bevorzugte cyanmethylierte Additive sind Verbindungen der Formel I, worin
- Z, m, n und p: die oben angegebenen Bedeutungen besitzen,
- R¹ und R²: unabhängig voneinander ausgewählt sind unter einen Wasserstoffatom und einem Alkylrest, insbesondere einem C₁-C₁₀-Alkylrest,
- R³: für ein Wasserstoffatom, einen Alkylrest, insbesondere einen C₁-C₁₀-Alkylrest, oder einen Cyanorest der Formel IVb steht, und
- R⁴ und R⁵: unabhängig voneinander für ein Wasserstoffatom oder einen C₁-C₆-Alkylrest stehen.

Besonders bevorzugt sind solche Additive, worin Z ein aus Isobuteneinheiten aufgebauter Rest mit einem zahlenmittleren Molekulargewicht von 800 - 1500 ist, n für 0 oder 1 steht, und Y für eine Gruppe der Formel

- CR¹R² - CN

steht, worin R¹ und R² unabhängig voneinander für ein Wasserstoffatom oder eine C₁-C₁₀-Alkylgruppe stehen.

Eine weitere bevorzugte Ausführungsform der Erfindung betrifft Diketenderivate der allgemeinen Formel Ia 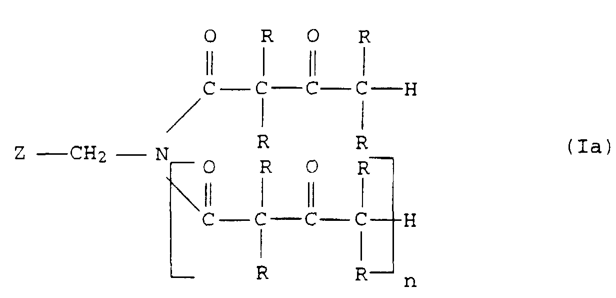 worin Z, R und n die oben angegebenen Bedeutungen besitzen.

Die Reste R stehen vorzugsweise unabhängig voneinander für ein Wasserstoffatom, einen geradkettigen oder verzweigten, gegebenenfalls substituierten C₁-C₃₀-Alkyl-, C₂-C₃₀-Alkenyl- oder C₂-C₃₀-Alkinylrest, oder einen gegebenenfalls substituierten C₃-C₈-Cycloalkyl-, Phenyl-, Naphthyl- oder Phenyl-C₁-C₁₂-alkyl- oder Naphthyl-C₁-C₁₂-alkylrest, der gegebenenfalls ein oder mehrere Heteroatome trägt.

Besonders bevorzugt sind Verbindungen der Formel Ia, worin Z ein zahlenmittleres Molekulargewicht von 800 bis 1500 besitzt, n für 0 oder 1 steht und die Reste R gleich sind und jeweils für ein Wasserstoffatom oder einen C₁-C₃₀-Alkylrest stehen.

Der Polyalkylrest Z in Verbindungen der allgemeinen Formel (I) ist vorzugsweise erhältlich durch Homo- oder Copolymerisation von geradkettigen oder verzweigten C₂-C₃₀-Alkenen, wobei C₂-C₆-Alkene und insbesondere C₂-C₄-Alkene bevorzugt sind. Besonders bevorzugte C₂-C₄-Alkene sind 1-Alkene, wie Propylen, 1-Buten, Isobuten. Beispielsweise kann Z von einem Copolymer aus 1-Buten und Isobuten abgeleitet sein, wobei Z ebenfalls ein mittleres Molekulargewicht von 800 bis etwa 1500 aufweisen kann.

Erfindungsgemäß verwendbare Alkylreste sind geradkettige oder verzweigte, gesättigte Kohlenstoffketten mit 1 bis 30 Kohlenstoffatomen. Beispielsweise können folgende Reste genannt werden: C₁-C₆-Alkylreste, wie Methyl, Ethyl, n-Propyl, i-Propyl, n-Butyl, sec.-Butyl, i-Butyl, t-Butyl, n-Pentyl, sec.-Pentyl, i-Pentyl, n-Hexyl, 1-,2-oder 3-Methyl-pentyl; längerkettige Alkylreste, wie unverzweigtes Heptyl, Octyl, Nonyl, Decyl, Undecyl, Lauryl, Tridecyl, Myristyl, Pentadecyl, Palmityl, Heptadecyl, Stearyl, Nonadecyl, Arachinyl, Behenyl, Lignoceryl, Ceryl und Myricyl, sowie die ein- oder mehrfach verzweigten Analoga davon. Bevorzugte langkettige Reste sind Lauryl, Myristyl, Palmityl, Stearyl und Arachinyl.

Erfindungsgemäß verwendbare Alkenylreste sind geradkettige oder verzweigte Kohlenstoffketten mit wenigstens einer Kohlenstoff-Kohlenstoff-Doppelbindung und mit 2 bis 30 Kohlenstoffatomen. Als Beispiele für einfach ungesättigte C₂-C₃₀-Alkenylreste können genannt werden C₂-C₆-Alkenylreste, wie Vinyl, Allyl, 1-Propenyl, Isopropenyl, 1-, 2- oder 3-Butenyl, Methallyl, 1,1-Dimethylallyl, 1-, 2-, 3-, 4- oder 5-Hexenyl; längerkettige Reste, wie unverzweigtes Heptenyl, Octenyl, Nonenyl, Decenyl, Undecenyl, Dodecenyl, Tridecenyl, Pentadecenyl, Palmitoleyl, Icosenyl und Triacontenyl, und die verzweigten Analoga davon, wobei die Doppelbindung in beliebiger Position auftreten kann. Erfindungsgemäß mitumfaßt werden sowohl die cis- als auch die trans-Isomeren obiger C₂-C₃₀-Alkenylreste. Ein bevorzugter einfach ungesättigter langkettiger Rest ist der Oleylrest.

Erfindungsgemäß verwendbare Alkinylreste sind geradkettige oder verzweigte Kohlenstoffketten mit wenigstens einer Kohlenstoff-Kohlenstoff-Dreifachbindung und 2 bis 30 Kohlenstoffatomen. Beispiele umfassen Ethinyl, 1- oder 2-Propinyl, 1-, 2- oder 3-Butinyl, sowie die entsprechenden Alkinylanaloga oben genannter Alkenylreste.

Beispiele für erfindungsgemäß verwendbare Cycloalkylreste sind C₃-C₈-Cycloalkylreste, wie Cyclopropyl, Cyclobutyl, Cyclopentyl, Cyclohexyl, Cycloheptyl, Cyclooctyl, Cyclopropylmethyl, Cyclopropylethyl, Cyclopropylpropyl, Cyclobutylmethyl, Cyclobutylethyl, Cyclopentylethyl, Cyclopentylpropyl und dergleichen.

Beispiele für erfindungsgemäß verwendbare Arylreste sind Phenyl und Naphthyl.

Erfindungsgemäß verwendbare Arylalkylreste sind insbesondere Phenyl-C₁-C₁₀-alkyl und Naphthyl-C₁-C₁₀-alkyl, wobei der C₁-C₁₀-Alkylteil wie oben angegeben definiert ist.

Die erfindungsgemäß verwendeten Cycloalkyl-, Aryl- und Arylalkylgruppen können gegebenenfalls 1 oder mehrere, wie z.B. 1 bis 4 Heteroatome, wie O, S und N enthalten, wobei Sauerstoff und Stickstoff als Heteroatom bevorzugt ist. Beispiele für cyclische Heteroalkylreste sind Tetrahydrofuranyl, Piperidinyl, Piperazinyl und Morpholinyl. Beispiele für Heteroarylgruppen sind 5- oder 5 6-gliedrige aromatische Ringsysteme, die 1 bis 4 der genannten Heteroatome umfassen, wie z.B. Furyl, Pyrrolyl, Imidazolyl, Pyrazolyl, Oxazolyl, Isoxazolyl, Oxadiazolyl, Tetrazolyl, Pyridyl, Pyrimidinyl, Pyrazinyl, Pyradizinyl, Triazinyl, Tetrazinyl und dergleichen.

Erfindungsgemäß anwendbare geradkettige oder verzweigte Alkylenreste umfassen geradkettige C₁-C₁₀-Alkylenreste, wie z.B. Ethylen, Propylen, Butylen, Pentylen und Hexylen, sowie verzweigte C₁-C₁₀-Alkylenreste, wie z.B. 1,1-Dimethylethylen, 1,3-Dimethylpropylen, 1-Methyl-3-ethyl-propylen, 2,3-Dimethylbutylen, 1,3-Dimethylbutylen, 1,1-Dimethylbutylen, 1,2-Dimethylpentylen und 1,3-Dimethylhexylen.

Beispiele für erfindungsgemäß geeignete Substituenten sind C₁-C₆-Alkyl, C₁-C₆-Alkyloxy, C₂-C₆-Alkenyl, C₁-C₆-Alkanoyl, wie z. B. Acetyl und Propionyl, Nitro und Amino.

Gegenstand der Erfindung ist außerdem ein Verfahren zur Herstellung von Verbindungen der allgemeinen Formel I, wobei man ein Polyalkylamin der allgemeinen Formel VI 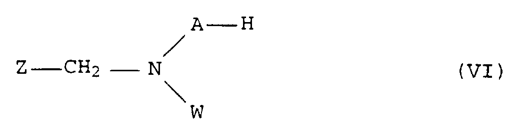 worin
- Z: die oben angegebenen Bedeutungen besitzt,
- W: für ein Wasserstoffatom oder -A-H steht, und
- A: für einen Alkyleniminrest der Formel VII 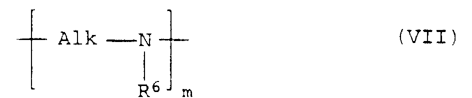 steht, worin Alk und m die oben angegebenen Bedeutungen besitzen und R⁶ für ein Wasserstoffatom, einen geradkettigen oder verzweigten Alkyl-, Alkenyl- oder Alkinylrest oder einen Arylrest steht, entweder
a₁) mit mindestens einem Diketen der allgemeinen Formel IX 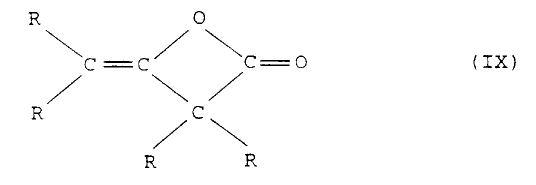 worin R die oben angegebenen Bedeutungen besitzt, in einem inerten Lösungsmittel umsetzt, um eine Verbindung der Formel I zu erhalten, die wenigstens einen Rest Y mit der Formel IIa aufweist; oder
a₂) mit Blausäure oder einem Salz davon und mindestens einer Verbindung der allgemeinen Formel VIII 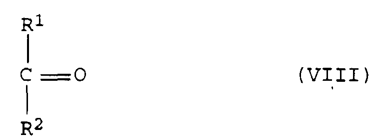 worin R¹ und R² die oben angegebenen Bedeutungen besitzen, um eine Verbindung der Formel I zu erhalten, die wenigstens einen Rest Y mit der Formel IIb aufweist.

Die Umsetzung nach Verfahren a₂) erfolgt vorzugsweise in einem wäßrigen Medium in Gegenwart eines Phasentransfer-Katalysators.

Steht in dem nach Verfahren a₁) oder a₂) erhaltenen Reaktionsprodukt W für ein Wasserstoffatom, so kann man dieses gegebenenfalls in herkömmlicher Weise durch eine Gruppe der Formel V 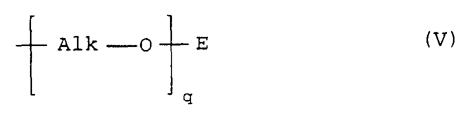 worin Alk, E und q die oben angegebenen Bedeutungen besitzen, substituieren.

Die Derivatisierung der Polyalkylamine mit Diketenen nach Verfahren a₁) zu den erfindungsgemäßen Diketenverbindungen erfolgt in an sich bekannter Art und Weise, indem das Polyalkylamin in Substanz oder in einem inerten Lösungsmittel, unter Kühlung, bei Raumtemperatur oder aber auch bei erhöhter Temperatur - je nach Reaktivität der Reaktionspartner - mit dem Diketen versetzt wird. Beispiele für geeignete Lösungsmittel umfassen schwefel- und chlorfreie Lösungsmittel, wie höhersiedende Kohlenwasserstoffe, wie z.B. n-Hexan, n-Octan, n-Decan oder Isododecan, oder dipolar aprotische Lösungsmittel, wie wasserfreies Tetrahydrofuran. Vorzugsweise löst man das Polyalkylamin in einem geeigneten Lösungsmittel und gibt das Diketen, gegebenenfalls gelöst in dem gleichen Lösungsmittel, tropfenweise unter Rühren hinzu. Die erhaltenen Reaktionsprodukte können, gegebenenfalls nach Abdestillieren des Lösungsmittels oder Entfernen überschüssiger Reagenzien, ohne weitere Reinigung verwendet werden.

Die Cyanmethylierung von Aminen erfolgt gemäß Stand der Technik üblicherweise nach der sogenannten "Strecker-Synthese" (siehe beispielsweise: Strecker, Ann. Chem. 75, 27 (1850); Kendall, Mc Kenzie, Org. Synth., 9, 4 (1929); H. Bucherer, A. Grolée, Chem. Ber. 39, 992 (1906); E. Knoevenagel, Chem. Ber. 37, 402 (1904); DE 2 107 757; DE 2 624 891) durch Umsetzung des Amins mit wäßrigem Formaldehyd und Blausäure. Eine Umsetzung von Polyisobutenaminen obiger Formel VI mit Blausäure beziehungsweise Blausäuresalzen und Formaldehyd beziehungsweise Paraformaldehyd führt, auch unter Zusatz von Lösungsvermittlern, nicht zur gewünschten Umsetzung. Überraschenderweise wurde jedoch erfindungsgemäß festgestellt, daß die Herstellung der erfindungsgemäßen cyanmethylierten Additive nach Verfahren a₂) durch Zusatz eines Phasentransferkatalysators in überraschend guter Ausbeute gelingt.

Die nucleophile Substitution von halogenierten Kohlenwasserstoffverbindungen mit Natriumcyanid unter Phasentransferkatalyse ist allgemein bekannt (C. M. Starks, J. A. Chem. Soc. 93, 195 (1971)), ebenso wie die Hydrocyanierung von Carbonylverbindungen mit Phasentransferkatalysatoren (C. L. Liotta, A. M. Dabdoub, L. H. Zalkow, Tetrahedron Lett., 1117 (1977)). Neu ist allerdings der Einsatz von Phasentransferkatalysatoren bei der Cyanomethylierung von hydrophoben Aminen. Überraschend dabei ist, daß die Reaktion beschleunigt wird, obwohl Formaldehyd in wäßriger Lösung zugesetzt wird.

Geeignete Phasentransfer-Katalysatoren zur Durchführung des erfindungsgemäßen Verfahrens a₂) umfassen quaternäre Ammonium- und Phosphoniumsalze, wobei jedoch quaternäre Ammoniumsalze bevorzugt sind. Beispiele für verwendbare Phasentransferkatalysatoren sind Benzyltriethylammoniumchlorid, Tetrabutylammoniumbromid, Methyltricaprylammoniumchlorid und Methyltributylammoniumchlorid sowie die entsprechenden halogenfreien Formen dieser Verbindungen.

Das erfindungsgemäße Herstellungsverfahren a₂) wird vorzugsweise so durchgeführt, daß man das Polyalkylamin der Formel VI in einem geeigneten Lösungsmittel, wie z.B. Tetrahydrofuran oder anderen dipolar aprotischen Lösungsmitteln, löst und diese Lösung mit dem Phasentransfer-Katalysator versetzt. Dann gibt man eine wässrige Cyanidlösung hinzu, in welcher der Ketoverbindung der Formel VIII gelöst ist. Das Reaktionsgemisch erwärmt man auf etwa 40-70 °C und hält dabei den pH-Wert der Lösung etwa auf 8 bis 9. Nach beendeter Reaktion trennt man die organische Phase ab und isoliert daraus das gewünschte Additiv.

Werden beim erfindungsgemäßen Herstellungsverfahren Gemische von Ketoverbindungen der allgemeinen Formel VIII eingesetzt, so enthalten die erfindungsgemäßen Additive gleichzeitig verschiedene Cyano-Endgruppen der Formel IIb.

Die Polyalkylamine der allgemeinen Formel VI können durch Hydroformylierung reaktiver Polyalkene und anschließende reduktive Aminierung des Oxoproduktes hergestellt werden. Die reaktiven Polyalkene mit einem mittleren Molekulargewicht von 500 bis 40000, sind Homo- oder Copolymere aus geradkettigen oder verzweigten C₂-C₃₀-Alkenen, vorzugsweise C₂-C₆-Alkenen, insbesondere C₂-C₄-Alkenen. Reaktive Polyalkene umfassen ungesättigte Polymere hoher chemischer Homogenität, wobei mehr als 10 % der Doppelbindungen alpha-ständig sind. Eine Möglichkeit zur Herstellung reaktiver Polyalkene ist in der DE 27 02 604 offenbart. Insbesondere sind solche reaktive Polyalkene bevorzugt, die aus 1-Alkenen, wie insbesondere Propylen, 1-Buten, Isobuten oder Gemischen davon, hergestellt sind.

Als Polyalkylamine der allgemeinen Formel VI kommen auch in Betracht Amine gemäß EP 0 244 616 und EP 0 695 338, auf deren Inhalt hiermit Bezug genommen wird. Die EP 0 244 616 beschreibt insbesondere solche Polyalklamine, worin der Rest Z abgeleitet ist von Isobuten und bis zu 20 Gew.-% n-Buten, wobei das Molekulargewicht des Polyisobutenrests bei etwa 300 bis etwa 5000 liegen kann. Die EP 0 695 338 beschreibt insbesondere solche Polyalkylamine, worin der Rest Z abgeleitet ist von einem oder mehreren 1-n-Alkenen mit 3 - 6 Kohlenstoffatomen und bis zu 50 Gew.-% Ethen, wobei Z etwa 20 - 400 Kohlenstoffatome aufweisen kann.

Wesentliches Kriterium für alle erfindungsgemäß brauchbaren Polyalkylamine ist aber, daß sie mindestens eine primäre oder sekundäre Amingruppe enthalten, die durch Cyanmethylierung oder durch Umsetzung mit Diketenen, wie oben beschrieben, derivatisiert werden kann.

Erfindungsgemäß besonders bevorzugt werden Additive ausgehend von Polybutyl- und Polyisobutylaminen der Formel VI hergestellt, die aus der EP 0 244 614 bekannt sind.

Gegenstand der Erfindung sind außerdem Schmierstoffzusammensetzungen, welche wenigstens ein erfindungsgemäßes Polyalkylaminderivat gemäß obiger Definition, gegebenenfalls in Kombination mit weiteren üblichen Schmierstoffzusätzen enthalten. Beispiele für übliche Zusätze sind Korrosionsinhibitoren, Verschleißschutzadditive, Viskositätsverbesserer, Detergenzien, Antioxidantien, Antischaummittel, Schmierfähigkeitsverbesserer und Stockpunktverbesserer. Die erfindungsgemäßen Verbindungen sind üblicherweise in Mengen von 1 bis 15 Gew.-%, vorzugsweise 0,5 bis 10 Gew.-%, bezogen auf das Gesamtgewicht der Zusammensetzung enthalten.

Beispiele für erfindungsgemäß hergestellte Schmierstoffe umfassen Öle und Fette für Kraftfahrzeuge und industriell eingesetzter Antriebsaggregate, wie insbesondere Motorenöle, Getriebeöle und Turbinenöle.

Die Erfindung betrifft außerdem Kraftstoffzusammensetzungen, wie z.B. Kraftstoffe für Otto- und Dieselmotoren, welche die erfindungsgemäßen Additive enthalten. Die erfindungsgemäßen Verbindungen dienen darin insbesondere als Detergenzien zur Reinhaltung des Kraftstoffeinlaßsystems. Aufgrund ihrer dispergierenden Eigenschaften üben sie einen positiven Einfluß auf den Motorschmierstoff aus, in welchen sie während des Betriebs gelangen können.

Zur Prüfung der erfindungsgemäßen Produkte hinsichtlich ihrer Dispergatoreigenschaften kann ein "Tüpfeltest", wie er zum Beispiel von A. Schilling in "Les Huiles pour Moteurs et la Graissage des Moteurs", Vol. 1, 1962, Seite 89f. in etwas modifizierter Form beschrieben ist, herangezogen werden.

Die erfindungsgemäßen Polyalkylamin-Derivate werden handelsüblichen Kraftstoffen in Konzentration von 20 bis 5000 mg/kg, vorzugsweise 50 bis 1000 mg/kg Kraftstoff zudosiert. Die erfindungsgemäßen Additive können gegebenenfalls auch zusammen mit anderen bekannten Additiven zugesetzt werden.

Während in Schmierstoffzusammensetzungen vorzugsweise solche erfindungsgemäßen Additive verwendet werden, worin Z ein zahlenmittleres Molekulargewicht von 2000 - 40000 aufweist, eignen sich für die Verwendung als Kraftstoffadditiv insbesondere solche Verbindungen, worin Z ein zahlenmittleren Molekulargewicht von 500 - 5000, vorzugsweise von 500 - 2500 und insbesondere von 800 - 1500 aufweist.

Schließlich betrifft die vorliegende Erfindung Additivgemische, welche in konzentrierter Form wenigstens eine erfindungsgemäße Verbindung in Kombination mit anderen Kraftstoffadditiven, insbesondere Detergenzien und Dispergatoren, enthalten. Besonders bevorzugt ist eine Kombination mit beispielsweise aus der US 4,832,702 bekannten Polyisobutylaminen.

Als Detergens-Komponente kann in den erfindungsgemäßen Additivgemischen prinzipiell jedes bekannte der hierfür geeigneten Produkte eingesetzt werden, wie sie z.B. bei J. Falbe, U. Hasserodt, Katalysatoren, Tenside und Mineralöladditive, G. Thieme Verlag Stuttgart 1978, S. 221 f. oder bei K. Owen, Gasoline and Diesel Fuel Additives, John Wiley & Sons 1989, S. 23 ff, beschrieben sind.

Vorzugsweise verwendet man N-haltige Detergenzien, z.B. Verbindungen, die eine Amin- oder Amid-Gruppe enthalten. Insbesondere geeignet sind Polyisobutylamine gemäß EP 0 244 616, Ethylendiamintetraessigsäureamide und/oder -imide gemäß EP 0 188 786 oder Polyetheramine gemäß EP 0 356 725, wobei auf die Definitionen in diesen Literaturstellen Bezug genommen wird. Die dort beschriebenen Produkte verfügen herstellungsbedingt ebenfalls über den Vorteil, chlor- bzw. chloridfrei zu sein.

Andererseits können diese Additive auch mit Trägerölen kombiniert werden. Insbesondere eignen sich Trägeröle auf Polyalkylenglykolbasis, z.B. entsprechende Ether und/oder Ester, wie sie in der US 5 004 478 oder der DE 38 38 918 A1 beschrieben sind. Auch Polyoxalkylenmonoole mit Kohlenwasserstoffendgruppen (US 4 877 416) oder Trägeröle, wie sie in der DE 41 42 241 offenbart sind, können eingesetzt werden.

Die vorliegende Erfindung wird anhand folgender Ausführungsbeispiele näher erläutert.

### Beispiel 1

### Herstellung von cyanmethyliertem Polyisobutenamin

250 g Polyisobutenamin (M_{w} ca. 1000) werden mit 125 g Tetrahydrofuran und 250 g Wasser gemischt und mit 55 g Formaldehyd (30%ig) versetzt. Nach Zugabe von 0,57 g Benzyltriethylammoniumchlorid und 81,7 g 33%ig wäßriger Natriumcyanid-Lösung wird der pH-Wert der wäßrigen Lösung mit 56 g 50%ig wäßriger Schwefelsäure auf ca. 9 eingestellt. Unter starkem Rühren wird der pH-Wert während 2 h bei 60°C durch Zugabe von insgesamt 14 g 15%ige wäßriger Natronlauge im Bereich von 8 -9 gehalten. Nach dieser Zeit beträgt der Umsatz bezogen auf unumgesetztes Cyanid 89 %. Nach dem Erkalten wird die überstehende organische Phase abgetrennt und noch zweimal mit 300 g Wasser ausgeschüttelt. Nach Abziehen des Lösungsmittels im Vakuum verbleiben 244 g hochviskoses Produkt, entsprechend 90 % der Theorie. Man erhält ein Gemisch aus unsubstituiertem, monosubstituiertem und disubstituiertem Polyisobutenamin, wie durch NMR und Massenspektrometrie bestimmt.

### Beispiel 2 (Vergleichsbeispiel)

a) Zu einer Lösung von 500 g Polyisobutenamin in 1,5 1 Tetrahydrofuran werden bei 20°C nacheinander 110 g 30%iger wäßriger Formaldehyd und 30 g Blausäure getropft. Diese Mischung wird 35 h bei 60°C gerührt, nach dieser Zeit beträgt der Umsatz bezogen auf unumgesetztes Cyanid <20 %. Nach Abtrennung der organischen Phase und Abdestillieren des Lösungsmittels wird nur unumgesetztes Polyisobutenamin erhalten.
b) Zu einer Lösung von 250 g Polyisobutenamin in 100 g Tetrahydrofuran werden bei 20°C 15 g Blausäure getropft und nachfolgend bei der gleichen Temperatur eine Lösung von 22,5 g Trioxan in 50 g Tetrahydrofuran innerhalb 1 h zugetropft. Nach insgesamt 30 h bei 60°C wird das Lösungsmittel entfernt, dabei verbleibt laut NMR-Analyse Polyisobutenamin mit einem Substitutionsgrad von <20 %.

### Beispiel 3

### Umsetzung von Polyisobutylamin (PIBA) mit Distearyldiketen

Zu einer Lösung von 0,4 Mol PIBA (PIBA, hergestellt nach EP 0 244 616, NW ∼ 1041) in Isododecan wurden bei einer Temperatur von 40°C eine äquimolare Menge an Distearyldiketen (0,4 Mol) im Verlauf von 20 min. zugesetzt. Die so erhaltene Lösung wurde bei 40°C noch weitere 30 min. gerührt und dann auf Raumtemperatur abgekühlt. Man erhielt eine farblose Flüssigkeit.
Ausbeute: quantitativ
IR (Film, cm⁻¹): 3300 (N-H); 2950; 2925; 2845; 1705 (C=O); 1630 (O=C-NH-); 1455; 1390; 1350; 1220

### Beispiel 4

### Umsetzung von Polyisobutylamin mit Distearyldiketen

0,4 Mol PIBA (PIBA, hergestellt nach EP 0 244 616, NW ∼ 1041) wurde auf eine Temperatur von 60°C erhitzt. Anschließend wurden langsam 0,4 Mol Distearyldiketen zugesetzt. Die Reaktionsmischung wurde nach beendeter Zugabe noch 45 min nachgerührt und dann auf Raumtemperatur abgekühlt. Man erhielt ein farbloses, zähes Öl.
Ausbeute: quantitativ
IR (Film, cm⁻¹): 3300 (N-H); 2950; 2925; 2845; 1705 (C=O); 1630 (O=C-NH-); 1455; 1390; 1350; 1220

### Beispiel 5

### Umsetzung von PIBA mit Dioleyldiketen

Es wurde eine Lösung von 0,4 Mol PIBA (PIBA, hergestellt nach EP 0 244 616, MW ∼ 1041) in Isododecan bei 30°C vorgelegt. Unter kräftigem Rühren wurden langsam 0,4 Mol Dioleyldiketen zugetropft. Nach Ende der Zugabe wurde die Lösung noch 45 min. gerührt. Man erhielt eine hellgelbe Flüssigkeit.
Ausbeute: quantitativ
IR (Film, cm⁻¹): 3300 (N-H); 2950; 2925; 2845; 1705 (C=O); 1630 (O=C-NH-); 1455; 1390; 1350; 1220

### Beispiel 6

### Umsetzung von Polyisobutylamin (PIBA) mit Diketen

Zu einer Lösung von 207,8 g (0,20 Mol) Polyisobutylamin (PIBA, hergestellt nach EP 0 244 616, MW ∼ 1041) in 1000 ml Tetrahydrofuran (THF; wasserfrei) wurde bei 0°C unter Rühren während einer Stunde eine Lösung von 17,6 g (0,21 Mol) Diketen in 100 ml THF getropft. Die Reaktion verlief schwach exotherm. Der Rührvorgang wurde noch zwei Stunden bei 0°C fortgesetzt. Anschließend destillierte man das Lösungsmittel ab und entfernte verbleibende Spuren von THF und Diketen bei 50°C und 0,5 mbar. Zurück blieben 222,0 g (0,197 Mol) N-Acetoacetyl-polyisobutylamin als schwach gelbliches zähes Öl.
Ausbeute: nahezu quantitativ
Amin-Zahl: 0
IR (Film, cm⁻¹): 2950, 1650, 1470, 1390, 1365, 1230
13C-NMR (100 MHz, CDCl₃, Auszug): δ = 22,70 (q, CH-CH₃), 29,54 (q, H₃C-C=O), 37,75 (t, CH₂-CH₂-N), 49,51 (t, CH₂-CO-CH₃), 165,11 (HN-C=O), 204,76 (H₃C-C=O), 31,24 (q), 38,15 (s), 59,70 (t) (Polyisobutyl).
Zum Vergleich: ¹³C-NMR vom eingesetzten PIBA (100 MHz, CDCl₃, Auszug): δ = 23,06 (CH-CH₃), 26,55 (CH-CH₃), 40,27 (CH₂-NH₂), 44,14 (CH₂-CH₂-NH₂); 31,32 (q), 38,17 (s), 59,56 (t) (Polyisobutyl).

### Beispiel 7

### Motortest zur Prüfung der Wirkung als Einlaßsystemreiniger

Die Prüfung der erfindungsgemäßen Produkte als Krafstoffadditive, besonders hinsichtlich ihrer Eignung als Einlaßsystemreiniger, geschieht mit Hilfe von Motorentests, die in Prüfstand-Versuchen mit einem 1,2 1 Opel Kadett-Motor gemäß CEC F/04/A/87 durchgeführt werden. Eingesetzter Kraftstoff: Euro Super bleifrei. Die Ergebnisse sind in folgender Tabelle 1 zusammengefaßt.

**Tab.1:**

| Verringerung von Ablagerungen am Einlaßventil | | | | | | |
|---|---|---|---|---|---|---|
| Additiv aus Beispiel | Dosierung [mg/kg] | Einlaßventilablagerungen [mg]* | | | | |
| | | Ventile | 1 | 2 | 3 | 4 |
| 1 | 200 | | 0 (554) | 12 (343) | 0 (293) | 6 (484 |
| 6 | 200 | | 3 (277) | 2 (175) | 2 (183) | 2 (337) |
| 3 | 200 | | 8 (554) | 4 (343) | 3 (293) | 5 (484) |

| | | | | | | |
|---|---|---|---|---|---|---|
| * Werte in Klammern: Ablagerungen ohne Additivzusatz | | | | | | |

## Patentansprüche

1. Verbindungen der allgemeinen Formel I
Z―CH₂―NY₂₋ₙHₙ (I)
worin
n für 0 oder 1 steht;
Z für einen geradkettigen oder verzweigten Polyalkylrest mit einem zahlenmittleren Molekulargewicht von 500 bis 40 000 steht;
Y für einen Rest der Formel IIa oder IIb 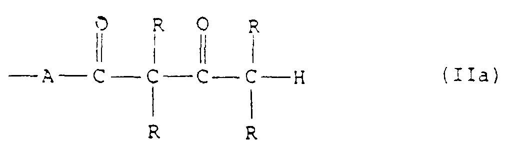 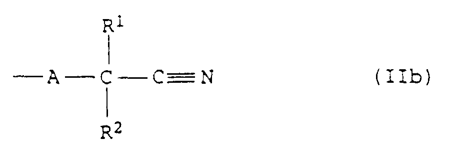 steht, worin
R, R¹ und R² gleich oder verschieden sind und unabhängig voneinander ausgewählt sind unter einem Wasserstoffatom, gegebenenfalls substituierten Alkyl-, Alkenyl-, Alkinylresten, und gegebenenfalls substituierten Cycloalkyl-, Aryl- oder Arylalkylresten, die gegebenenfalls ein oder mehrere Heteroatome aufweisen,
A für einen Alkyleniminrest der Formel III 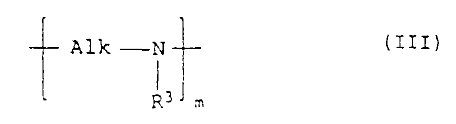 steht, worin
m für einen ganzzahligen Wert von 0 bis 10 steht;
Alk für einen geradkettigen oder verzweigten, gegebenenfalls substituierten Alkylenrest steht,
R³ für ein Wasserstoffatom, einen Alkyl-, Alkenyl-, Alkinyl- oder einen Arylrest steht oder, wenn Y für einen Rest der Formel IIa steht, für einen Ketorest der Formel IVa 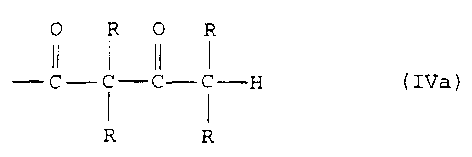 steht, worin die Reste R die oben angegebenen Bedeutungen besitzen,
oder, wenn Y für einen Rest der Formel IIb steht, für einen Cyanorest der Formel IVb 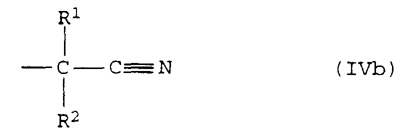 steht, worin R¹ und R² die oben angegebenen Bedeutungen besitzen;
oder,
wenn n für 0 steht, einer der Reste Y gegebenenfalls für einen Polyoxyalkylenrest der Formel V 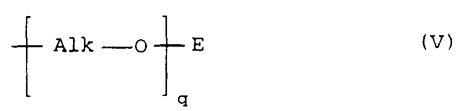 steht, worin
q für eine ganze Zahl von 1 bis 30 steht,
Alk wie oben definiert ist, und
E für ein Wasserstoffatom oder einen C₁-C₆-Alkylrest steht.

2. Verbindungen nach Anspruch 1 der Formel (I), worin Z und n die oben angegebenen Bedeutungen besitzten und Y für einen Rest der Formel IIc 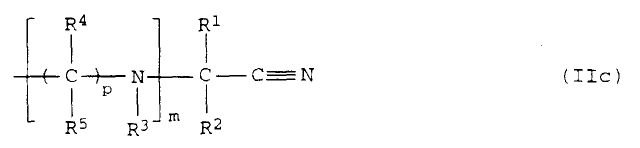 steht, worin
R¹, R², R³ und m die oben angegebenen Bedeutungen besitzen,
R⁴ und R⁵ gleich oder verschieden sind und die oben für R¹ und R² angegebenen Bedeutungen besitzen; und
p für einen ganzzahligen Wert von 2 bis 5 steht.

3. Verbindungen nach Anspruch 2, worin
Z, m, n und p die oben angegebenen Bedeutungen besitzen,
R¹ und R² unabhängig voneinander ausgewählt sind unter einen Wasserstoffatom und einem Alkylrest, insbesondere einem C₁-C₁₀-Alkylrest,
R³ für ein Wasserstoffatom, einen Alkylrest, insbesondere einen C₁-C₁₀-Alkylrest, oder einen Cyanorest der Formel IVb steht, und
R⁴ und R⁵ unabhängig voneinander für ein Wasserstoffatom oder einen C₁-C₆-Alkylrest stehen.

4. Verbindungen nach Anspruch 1 der Formel Ia 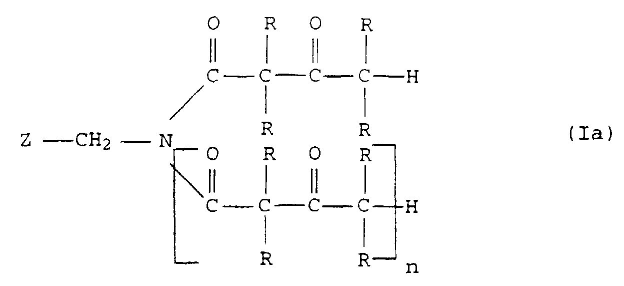 worin Z, R und n die oben angegebenen Bedeutungen besitzen.

5. Verbindungen nach Anspruch 1 oder 4, worin Z und n die oben angegebenen Bedeutungen besitzen und die Reste R unabhängig voneinander für ein Wasserstoffatom, einen geradkettigen oder verzweigten, gegebenenfalls substituierten C₁-C₃₀-Alkyl-, C₂-C₃₀-Alkenyl- oder C₂-C₃₀-Alkinylrest stehen oder einen gegebenenfalls substituierten C₂-C₈-Cycloalkyl-, Phenyl-, Naphthyl-, Phenyl-C₁-C₁₂-alkyl- oder Naphthyl-C₁-C₁₂-alkylrest bedeuten, der gegebenenfalls ein oder mehrere Heteroatome trägt.

6. Verbindungen nach einem der vorhergehenden Ansprüche, worin die Reste R die gleichen Bedeutungen besitzen und ausgewählt sind unter Wasserstoffatom und C₁-C₃₀-Alkyl.

7. Verbindungen nach einem der vorhergehenden Ansprüche, worin Z ein von mindestens einem geradkettigen oder verzweigten C₂-C₃₀-Alken, vorzugsweise C₂-C₆-Alken, insbesondere C₂-C₄-Alken oder Gemische davon abgeleiteter Polymerrest ist.

8. Verbindungen nach Anspruch 7, worin das Alken ein 1-Alken ist.

9. Verbindung nach Anspruch 8, worin das 1-Alken ausgewählt ist unter Propylen, 1-Buten und Isobuten.

10. Verbindungen nach Anspruch 9, worin Z abgeleitet ist von Polybuten oder Polyisobuten, oder einem Copolymerisat aus Isobuten und bis zu 20 Gew.-% n-Buten, und ein mittleres Molekulargewicht von 800 bis 1500 aufweist.

11. Verfahren zur Herstellung einer Verbindung nach einem der Ansprüche 1 bis 10, dadurch gekennzeichnet, daß man
a) ein Polyalkylamin der Formel VI 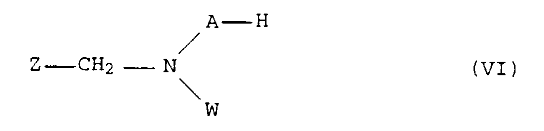 worin
Z die oben angegebenen Bedeutungen besitzt,
W für ein Wasserstoffatom oder -A-H steht, und
A für einen Alkyleniminrest der Formel VII 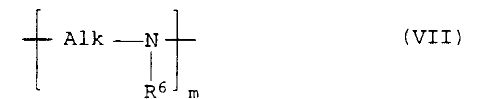 steht, worin Alk und m die oben angegebenen Bedeutungen besitzen und R⁶ für ein Wasserstoffatom, einen geradkettigen oder verzweigten Alkyl-, Alkenyl- oder Alkinylrest oder einen Arylrest steht, entweder
a₁) mit mindestens einem Diketen der allgemeinen Formel IX 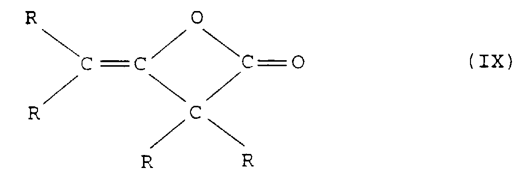 worin R die oben angegebenen Bedeutungen besitzt, in einem inerten Lösungsmittel umsetzt, um eine Verbindung der Formel I zu erhalten, die wenigstens einen Rest Y mit der Formel IIa aufweist; oder
a₂) mit Blausäure oder einem Salz davon und mindestens einer Verbindung der allgemeinen Formel VIII 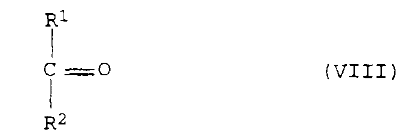 worin R¹ und R² die oben angegebenen Bedeutungen besitzen, umsetzt, um eine Verbindung der Formel I zu erhalten, die wenigstens einen Rest Y mit der Formel IIb aufweist;
und
b) wenn in dem gebildeten Produkt W für ein Wasserstoffatom steht, W gegebenenfalls durch eine Gruppe der Formel V 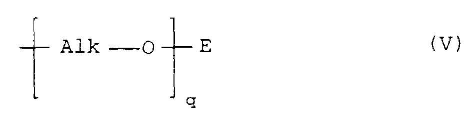 worin Alk, E und q wie oben definiert sind, substituiert.

12. Verfahren nach Anspruch 11, wobei die Umsetzung gemäß Stufe a₂) in einem wäßrigen Medium in Gegenwart eines Phasentransfer-Katalysators erfolgt.

13. Schmierstoffzusammensetzung, umfassend eine oder mehrere Verbindungen gemäß einem der Ansprüche 1 bis 10 in einer Gesamtmenge von 1 - 15 Gew.-%, bezogen auf das Gesamtgewicht der Zusammensetzung, gegebenenfalls in Kombination mit weiteren üblichen Schmierstoffadditiven.

14. Schmierstoffzusammensetzung nach Anspruch 13, umfassend mindestens eine Verbindung der allgemeinen Formel I, worin Z ein zahlenmittleres Molekulargewicht von 2000 bis 40000 aufweist.

15. Kraftstoffzusammensetzung, umfassend eine oder mehrere Verbindungen gemäß einem der Ansprüche 1 bis 10 in einer Gesamtkonzentration von 20 bis 5000 mg/kg Kraftstoff, gegebenenfalls in Kombination mit weiteren üblichen Kraftstoffadditiven.

16. Kraftstoffzusammensetzung nach Anspruch 15, umfassend mindestens eine Verbindung der allgemeinen Formel I, worin Z ein zahlenmittleres Molekulargewicht von 500 bis 2500 aufweist.

17. Additivgemisch für Kraft- oder Schmierstoffe, umfassend eine oder mehrere Verbindungen gemäß einem der Ansprüche 1 bis 10, gegebenenfalls in Kombination mit weiteren üblichen Additivkomponenten.

## Claims

1. A compound of the formula I
Z-CH₂-NY₂₋ₙHₙ (I)
where
n is 0 or 1,
Z is a straight-chain or branched polyalkyl radical having an average molecular weight of from 500 to 40,000,
Y is a radical of the formula IIa or IIb 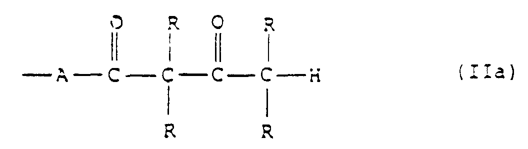 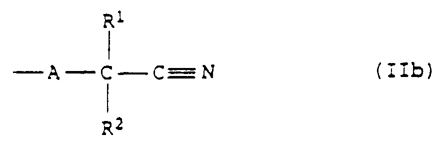 where
R, R₁ and R₂ are identical or different and, independently of one another, are selected from the group consisting of hydrogen, unsubstituted and substituted alkyl, alkenyl and alkynyl radicals and unsubstituted and substituted cycloalkyl, aryl and arylalkyl radicals which may contain one or more heteroatoms, and
A is an alkyleneimine radical of the formula III 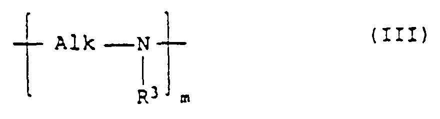 where
m is an integer from 0 to 10;
Alk is straight-chained or branched, unsubstituted or substituted alkylene,
R³ is hydrogen, alkyl, alkenyl, alkynyl or aryl or, if Y is a radical of the formula IIa, is a keto radical of the formula IVa 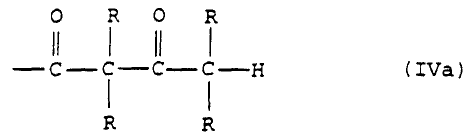 where the radicals R have the abovementioned meanings,
or, if Y is a radical of the formula IIb, is a cyano radical of the formula IVb 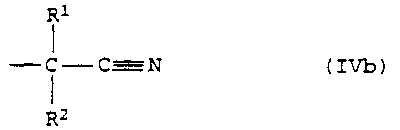 where R¹ and R² have the abovementioned meanings;
or,
if n is O, one of the radicals Y may be a polyoxyalkylene radical of the formula V 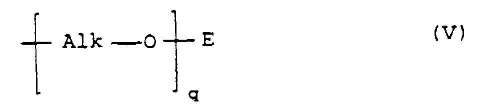 where
q is an integer from 1 to 30,
Alk is as defined above and
E is hydrogen or C₁-C₆-alkyl.

2. A compound as claimed in claim 1, of the formula (I), wherein Z and n have the abovementioned meanings and Y is a radical of the formula IIc 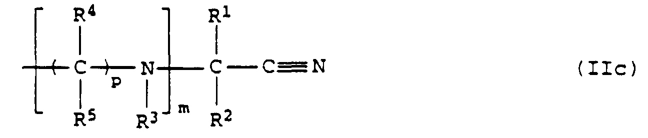 where
R¹, R², R³ and m have the abovementioned meanings,
R⁴ and R⁵ are identical or different and have the meanings stated above for R¹ and R² and
p is an integer from 2 to 5.

3. A compound as claimed in claim 2, wherein
Z, m, n and p have the abovementioned meanings,
R¹ and R², independently of one another, are selected from the group consisting of hydrogen and alkyl, in particular C₁-C₁₀-alkyl,
R³ is hydrogen, alkyl, in particular C₁-C₁₀-alkyl, or a cyano radical of the formula IVb and
R⁴ and R⁵, independently of one another, are each hydrogen or C₁-C₆-alkyl.

4. A compound as claimed in claim 1 of the formula Ia 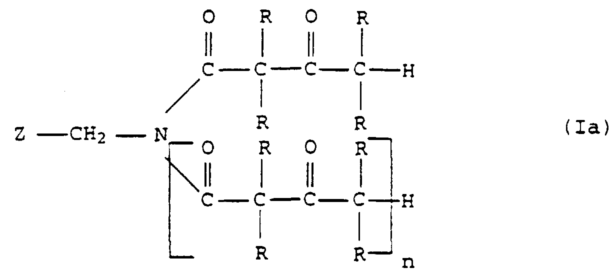 where Z, R and n have the abovementioned meanings.

5. A compound as claimed in claim 1 or 4, wherein Z and n have the abovementioned meanings and the radicals R, independently of one another, are each hydrogen, straight-chain or branched, unsubstituted or substituted C₁-C₃₀-alkyl, C₂-C₃₀-alkenyl or C₂-C₃₀-alkynyl or unsubstituted or substituted C₃-C₈-cycloalkyl, phenyl, naphthyl, phenyl-C₁-C₁₂-alkyl or naphthyl-C₁-C₁₂-alkyl radicals which may carry one or more heteroaroms.

6. A compound as claimed in any of the preceding claims, wherein the radicals R have the same meanings and are selected from hydrogen and C₁-C₃₀-alkyl.

7. A compound as claimed in any of the preceding claims, wherein Z is a polymer radical derived from at least one straight-chain or branched C₂-C₃₀-alkene, preferably C₂-C₆-alkene, in particular C₂-C₄-alkene, or a mixture thereof.

8. A compound as claimed in claim 7, wherein the alkene is a 1-alkene.

9. A compound as claimed in claim 8, wherein the 1-alkene is selected from propylene, 1-butene and isobutene.

10. A compound as claimed in claim 9, wherein Z is derived from polybutene or polyisobutene or a copolymer of isobutene and up to 20 % by weight of n-butene, and has an average molecular weight of from 800 to 1500.

11. A process for the preparation of a compound as claimed in any of claims 1 to 10, wherein
a) a polyalkylamine of the formula VI 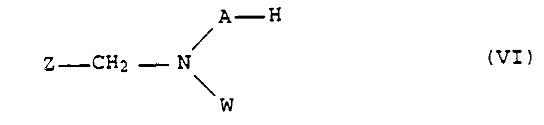 where
Z has the abovementioned meanings,
W is hydrogen or -A-H, and
A is an alkyleneimine radical of the formula VII 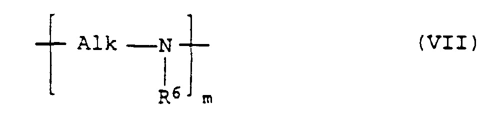 where Alk and m have the abovementioned meanings and R⁶ is hydrogen, straight-chain or branched alkyl, alkenyl or alkynyl or aryl, is reacted either
a₁) with at least one diketene of the formula IX 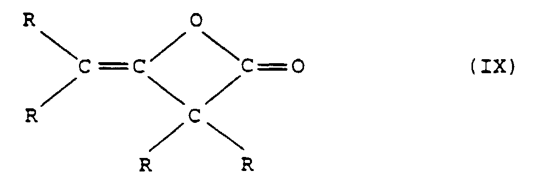 where R has the abovementioned meanings, in an inert solvent, in order to obtain a compound of the formula I which has at least one radical Y having the formula IIa; or
a2) with hydrocyanic acid or a salt thereof and at least one compound of the formula VIII 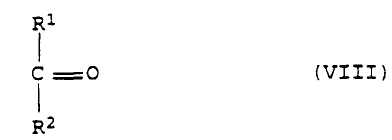 where R¹ and R² have the abovementioned meanings, in order to obtain a compound of the formula I which has at least one radical Y having the formula IIb;
and,
b) if W is hydrogen in the product formed, W is unsubstituted or substituted by a group of the formula V 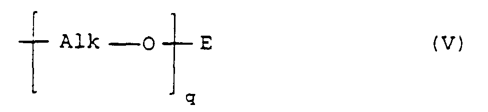 where Alk, E and q are as defined above.

12. A process as claimed in claim 11, wherein the reaction according to stage a₂) is carried out in an aqueous medium in the presence of a phase-transfer catalyst.

13. A lubricant composition comprising one or more compounds as claimed in any of claims 1 to 10 in a total amount of 1-15% by weight, based on the total weight of the composition, if required in combination with further conventional lubricant additives.

14. A lubricant composition as claimed in claim 13, comprising at least one compound of the formula I, where Z has a number average molecular weight of from 2000 to 40,000.

15. A fuel composition comprising one or more compounds as claimed in any of claims 1 to 10 in a total concentration of from 20 to 5000 mg/kg of fuel, if required in combination with further conventional fuel additives.

16. A fuel composition as claimed in claim 15, comprising at least one compound of the formula I, where Z has a number average molecular weight of from 500 to 2500.

17. An additive mixture for fuels or lubricants, comprising one or more compounds as claimed in any of claims 1 to 10, if required in combination with further conventional additive components.

## Revendications

1. Composés de formule générale I
Z ― CH₂ ― NH₂₋ₙHₙ (I)
dans laquelle
n est égal à 0 ou 1 ;
Z représente un groupe polyalkyle à chaîne droite ou ramifiée de poids moléculaire moyen, moyenne en nombre, 500 à 40 000 ;
Y représente un groupe de formule IIa ou IIb 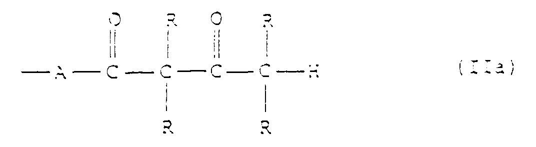 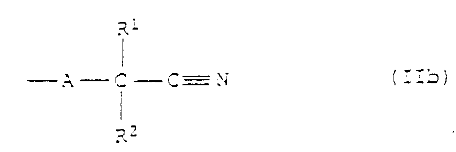 dans lesquelles
R, R¹ et R², ayant des significations identiques ou différentes, sont choisis chacun, indépendamment les uns des autres, parmi les atomes d'hydrogène, les groupes alkyle, alcényle et alcynyle éventuellement substitués et les groupes cycloalkyle, aryle ou arylalkyle éventuellement substitués, et qui contiennent le cas échéant un ou plusieurs hétéroatomes,
A représente un groupe alkylénimine de formule III 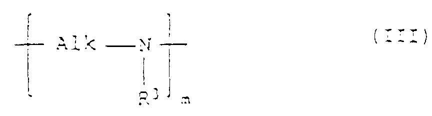 dans laquelle
m est un nombre entier allant de 0 à 10 ;
Alk représente un groupe alkylène à chaîne droite ou ramifiée éventuellement substitué,
R³ représente un atome d'hydrogène, un groupe alkyle, alcényle, alcynyle ou un groupe aryle, ou bien, lorsque Y représente un groupe de formule IIa, un groupe cétonique de formule IVa 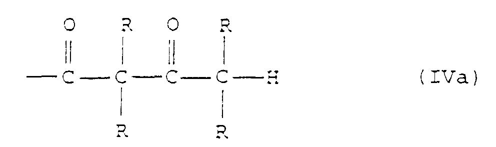
dans laquelle les symboles R ont les significations indiquées ci-dessus,
ou bien, lorsque Y représente un groupe de formule IIb, un groupe cyano de formule IVb 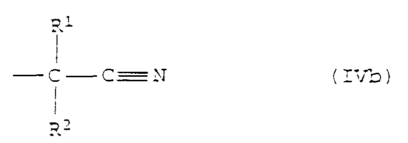 dans laquelle R¹ et R² ont les significations indiquées ci-dessus ;
ou bien, lorsque n est égal à 0, l'un des symboles Y représente le cas échéant un groupe polyoxyalkylène de formule V 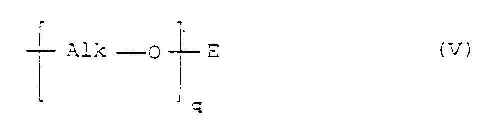 dans laquelle
q est un nombre entier allant de 1 à 30,
Alk a les significations indiquées ci-dessus et
E représente un atome d'hydrogène ou un groupe alkyle en C1-C6.

2. Composés selon la revendication 1, de formule I dans laquelle Z et n ont les significations indiquées ci-dessus et Y représente un groupe de formulé IIc 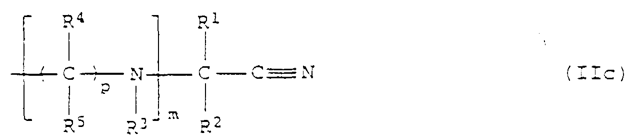 dans laquelle
R¹, R², R³ et m ont les significations indiquées ci-dessus,
R⁴ et R⁵, ayant des significations identiques ou différentes, ont les significations indiquées ci-dessus pour R¹ et R² ; et
p est un nombre entier allant de 2 à 5.

3. Composés selon la revendication 2, pour lesquels
Z, m, n et p ont les significations indiquées ci-dessus,
R¹ et R² sont choisis, indépendamment l'un de l'autre, parmi les atomes d'hydrogène et les groupes alkyle, plus spécialement les groupes alkyle en C1-C10,
R³ représente un atome d'hydrogène, un groupe alkyle, plus spécialement un groupe alkyle en C1-C10, ou un groupe cyano de formule IVb et
R⁴ et R⁵ représentent chacun, indépendamment l'un de l'autre, un atome d'hydrogène ou un groupe alkyle en C1-C6.

4. Composés selon la revendication 1, de formule Ia 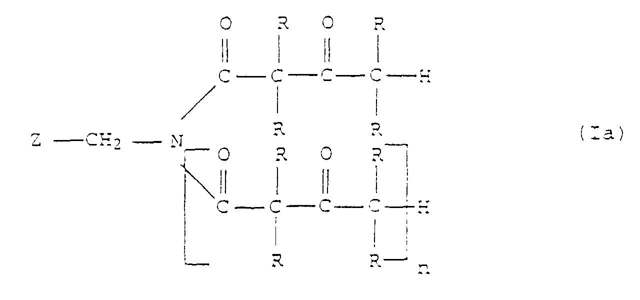 dans laquelle Z, R et n ont les significations indiquées ci-dessus.

5. Composés selon la revendication 1 ou 4, pour lesquels Z et n ont les significations indiquées ci-dessus et les symboles R représentent chacun, indépendamment les uns des autres, un atome d'hydrogène, un groupe alkyle en C1-C30, alcényle en C2-C30 ou alcynyle en C2-C30 à chaîne droite ou ramifiée et éventuellement substitué ou un groupe cycloalkyle en C3-C8, phényle, naphtyle, phényl-alkyle en C1-C12 ou naphtylalkyle en C1-C12 éventuellement substitué et qui contient le cas échéant un ou plusieurs hétéroatomes.

6. Composés selon l'une des revendications qui précèdent, pour lesquels les symboles R ont les mêmes significations et sont choisis parmi les atomes d'hydrogène et les groupes alkyle en C1-C30.

7. Composés selon l'une des revendications qui précèdent, pour lesquels Z est un radical polymère dérivant d'au moins un alcène à chaîne droite ou ramifiée en C2-C30, de préférence un alcène en C2-C6 et plus spécialement un alcène en C2-C4 ou un mélange de ces alcènes.

8. Composés selon la revendication 7, pour lesquels l'alcène est un 1-alcène.

9. Composé selon la revendication 8, pour lequel le 1-alcène est choisi parmi le propylène, le 1-butène et l'isobutène.

10. Composés selon la revendication 9, pour lesquels Z dérive d'un polybutène ou d'un polyisobutène ou d'un copolymère de l'isobutène et d'une proportion allant jusqu'à 20% en poids de n-butène, et il a un poids moléculaire moyen de 800 à 1500.

11. Procédé de préparation d'un composé selon l'une des revendications 1 à 10, caractérisé par le fait que l'on fait réagir
a) une polyalkylamine de formule VI 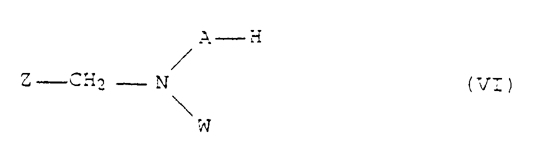 dans laquelle
Z a les significations indiquées ci-dessus,
W représente un atome d'hydrogène ou -A-H et
A représente un groupe alkylénimine de formule VII 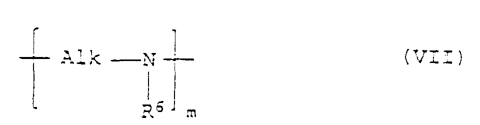 dans laquelle Alk et m ont les significations indiquées ci-dessus et R⁶ représente un atome d'hydrogène, un groupe alkyle, alcényle ou alcynyle à chaîne droite ou ramifiée ou un groupe aryle,
a₁) soit avec au moins un dicétène de formule générale IX 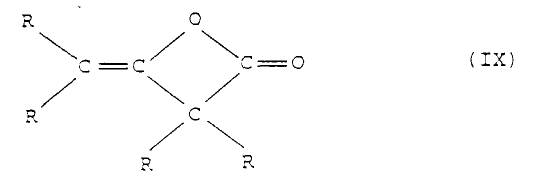 dans laquelle R a les significations indiquées ci-dessus, dans un solvant inerte, ce qui donne un composé de formule I contenant au moins un groupe Y de formule IIa ;
a₂) soit avec l'acide cyanhydrique ou un sel de cet acide et au moins un composé de formule générale VIII 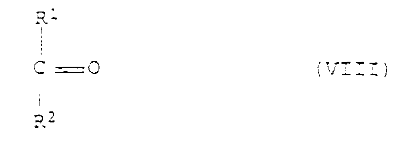 dans laquelle R¹ et R² ont les significations indiquées ci-dessus, ce qui donne un composé de formule I contenant au moins un groupe Y de formule IIb ;
et
b) lorsque, dans le produit formé, W représente un atome d'hydrogène, on remplace W le cas échéant par un groupe de formule V 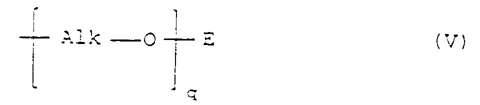 dans laquelle Alk, E et q ont les significations indiquées ci-dessus.

12. Procédé selon la revendication 11, dans lequel la réaction du stade a₂) est réalisée dans un milieu aqueux, en présence d'un catalyseur à transfert de phase.

13. Composition lubrifiante comprenant un ou plusieurs composés selon l'une des revendications 1 à 10 en quantité totale de 1 à 15% en poids, sur le poids total de la composition, éventuellement en combinaison avec d'autres additifs usuels pour lubrifiants.

14. Composition lubrifiante selon la revendication 13, comprenant au moins un composé de formule générale I dans laquelle Z a un poids moléculaire moyen, moyenne en nombre, de 2000 à 40 000.

15. Composition de carburant comprenant un ou plusieurs composés selon l'une des revendications 1 à 10 à la concentration totale de 20 à 5000 mg par kg de carburant, le cas échéant en combinaison avec d'autres additifs usuels pour carburants.

16. Composition de carburant selon la revendication 15, comprenant au moins un composé de formule I, dans laquelle Z présente un poids moléculaire moyen, moyenne en nombre, compris entre 500 et 2500.

17. Mélange additif pour carburant ou lubrifiant, comprenant un ou plusieurs composés selon l'une des revendications 1 à 10, éventuellement en combinaison avec d'autres additifs usuels.
